# EUROPEAN PATENT SPECIFICATION

(11) **EP 3 318 292 B1**
(45) Date of publication and mention of the grant of the patent: **24.07.2019**
(21) Application number: 15899437.6
(22) Date of filing: 29.10.2015
(51) Int. Cl.: A61M 1/02

(54) **SINGLE X-RAY SOURCE BLOOD IRRADIATION DEVICE AND IRRADIATION METHOD THEREOF**
BLUTBESTRAHLUNGSVORRICHTUNG MIT EINER EINZIGEN RÖNTGENQUELLE UND BESTRAHLUNGSVERFAHREN DAFÜR
DISPOSITIF D'EXPOSITION DU SANG À UNE SOURCE DE RAYONS X UNIQUE ET MÉTHODE D'EXPOSITION À UN RAYONNEMENT ASSOCIÉE

(30) Priority: 24.07.2015 CN 201510443398
(43) Date of publication of application: 09.05.2018
(73) Proprietor: Jiangsu Mocoto Medical Technology Co., Ltd., Suzhou, Jiangsu 215000 (CN)
(72) Inventor: DAI, Qiusheng, Suzhou Jiangsu 215000 (CN); XU, Ruxiang, Suzhou Jiangsu 215000 (CN); YANG, Lei, Suzhou Jiangsu 215000 (CN)
(74) Representative: 2K Patentanwälte Blasberg Kewitz & Reichel
(86) International application number: PCT/CN2015/093146
(87) International publication number: WO 2017/016085

(56) References cited:
- WO-A1-2017/035874
- CN-A- 1 736 495
- CN-A- 104 027 857
- CN-A- 104 324 425
- CN-A- 104 324 425
- CN-A- 104 701 119
- CN-A- 104 984 423
- CN-U- 204 972 435
- FR-B1- 2 492 210
- JP-A- 2005 224 401

## Description

### TECHNICAL FIELD

This invention relates generally to the technical field of blood irradiators, and more particularly relates to a blood irradiation apparatus with a single X-ray source and an irradiation method using the blood irradiation apparatus.

### BACKGROUND

Transfusion-associated graft-versus-host disease (TA-GVHD) is a fatal complication of blood transfusion, in which an immunocompromised or immunosuppressed patient is unable to destroy the immune-competent lymphocytes transfused into his bloodstream, allowing them to colonize and proliferate in his body and to identify his tissues and organs as non-self substances and set them as the target of immune attack and destruction. TA-GVHD lacks specific clinical manifestations and so can be very easily undiagnosed and misdiagnosed.

Studies have shown that the number of lymphocytes input to the recipient needs to exceed 107/kg to induce TA-GVHD, and should the number be less than 105/kg, TA-GVHD wouldn't be caused. But it has been reported that only 104/kg of lymphocytes could trigger TA-GVHD in children with immunodeficiency. In short, the higher the number of the donor's lymphocytes that are received, the more serious the condition and the higher the mortality rate.

Irradiation of blood or blood products before transfusion is at present considered the only effective way to prevent TA-GVHD. The underlying mechanism is that lymphocytes are sensitive to radiation so that by using the appropriate dose of radiation exposure, the immunocompetent lymphocytes can be inactivated and thus will lose their proliferative capacity, while the functions of erythrocytes and platelets as well as the activity of coagulation factors will not be affected. Now the application rate of blood irradiation in developed countries has reached 30% to 40%.

A blood irradiator is a medical device that uses radiation to irradiate blood or blood products, in order to inactivate lymphocytes and prevent TA-GVHD in blood transfusions.

By radiation sources, blood irradiators can be divided into two categories, one of which uses Y-rays generated by radioactive sources (mainly Cs-137, Co-60) to irradiate blood or blood products, while the other uses X-rays generated by a X-ray tube to irradiate blood or blood products. Blood irradiators using radioactive sources, however, need to be equipped with radioactive isotope sources with an activity of hundreds to thousands of curies, resulting in a potential risk of nuclear leakage. While there is no risk of nuclear leakage for the blood irradiators using X-ray sources and no radiation will be generated once the equipment is powered off, the blood irradiators using X-ray sources may have disadvantages of low energy of X-rays and uneven irradiation with a single source and so cannot meet the requirements on radiation uniformity specified in the blood irradiator standards (the national standards require the non-uniformity of irradiation of blood irradiators be lower than 20%). The energy of X-ray photons generated by an ordinary X-ray source is generally below 200 keV, so during the irradiation of blood or blood products, the X-ray photons can be easily absorbed by shallow blood so that the number of X-ray photons reaching the depth would rapidly decrease.

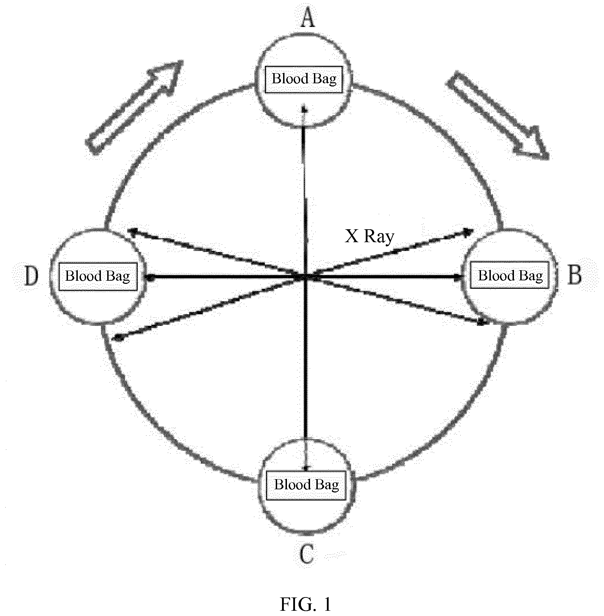

In order to overcome the shortcomings of existing blood irradiators using a single X-ray source, China Patent Publication No. CN104324425A, entitled "Blood Irradiator," discloses a blood irradiator which uses the movement of a cavity during the rotation of a turntable to alternately irradiate the upper and lower surfaces of a blood bag. Referring to FIG. 1, for example, X-rays are incident from below the blood bag at position A, while when C is reached, the X-rays become incident from above the blood bag. In this way, the uniformity of irradiation of the blood or blood products in the blood bag is improved, but the duration of irradiation was forced to double, which is therefore inconvenient.

### SUMMARY

The invention is defined in the appended claims. Where in the following the word invention is used and/or features are presented as optional this should be interpreted in such a way that protection is sought only for the invention as claimed. In view of the above problems, it is an object of this invention to provide a blood irradiation apparatus with a single X-ray source, the blood irradiation apparatus comprising a framework and an X-ray tube disposed on the framework. A blood bag receiver may be arranged below the X-ray tube. The blood bag receiver may be composed of a bottom plate, and an outer wall and inner wall arranged on the bottom plate. An annular blood bag accommodating cavity may be enclosed between the outer and inner walls and may be used for placing at least one blood bag to be irradiated. The blood bag may be filled with blood or a blood product. The bottom plate of the blood bag receiver may be movably disposed on the framework via a rotating shaft, and the rotating shaft may be connected with an output shaft of a rotating motor via a transmission.

The outer wall and the inner wall may run parallel to each other, and both the outer wall and the inner wall may be tilted relative to the bottom plate.

The outer wall and the inner wall may be separated by a distance that matches a thickness of the blood bag.

The blood bag receiver may be a truncated cone or a truncated pyramid.

The inner wall of the blood bag receiver may be made of a radiolucent material that is permeable to X rays.

The framework may be provided with a dose monitoring device disposed below the blood bag receiver and used for detecting an absorbed dose of the at least one blood bag.

The framework may be provided with a high-voltage power supply for supplying power to the X-ray tube. Each of the high-voltage power supply and the X-ray tube may be provided with a heat dissipating device.

The present invention also provides an irradiation method using the above-described blood irradiation apparatus with a single X-ray source. The method may comprise the following operations.

After at least one blood bag is placed in the blood bag accommodating cavity, the X-ray tube may start to work. As soon as the X-ray tube starts to work, the rotating motor may drive the blood bag receiver to rotate in a same direction at increasing speeds. An instantaneous angular velocity of the blood bag receiver may be detected, and when the angular velocity of the blood bag receiver reaches a predetermined speed, the blood bag receiver may start to decelerate. Then when the angular velocity of the blood bag receiver decreases to zero, the blood bag receiver may rotate at increasing speeds in a direction opposite to the direction of the previous rotation, and the instantaneous angular velocity of the blood bag receiver may again be detected, until predetermined stopping conditions are reached when the blood bag receiver will stop rotating and the X-ray tube be turned off.

Controlling the blood bag receiver to stop rotating and the X-ray tube to turn off when the predetermined stopping conditions are reached may comprise: detecting an absorbed dose of the at least one blood bag, and stopping the rotation of the blood bag receiver and turning off the X-ray tube when the absorbed dose of the at least one blood bag reaches a predetermined dose.

Controlling the blood bag receiver to stop rotating and the X-ray tube to turn off when the predetermined stopping conditions are reached may comprise: detecting a duration of rotation or a number of rotations of the blood bag receiver, and stopping the rotation of the blood bag receiver and turning off the X-ray tube when the duration of rotation or the number of rotations reaches a predetermined value.

According to this invention, the X-ray tube is surrounded by multiple blood bags to make full use of the X-rays generated by the X-ray tube, so that the utilization rate is improved. In addition, a rotating motor is used to rotate the blood bag receiver back and forth to force the blood or blood product in each blood bag to flow about, so that the blood or blood product in various local regions can exchange their positions, thereby improving the uniformity of radiation of the blood or blood product.

### BRIEF DESCRIPTION OF THE DRAWINGS

FIG. 1 is a schematic diagram illustrating the working principle of the prior art.
FIG. 2 is a schematic diagram of the overall structure of a blood irradiation apparatus in accordance with this invention.
FIG. 3 is a schematic diagram illustrating a structure of a blood bag receiver of a blood irradiation apparatus in accordance with this invention.
FIG. 4 is a schematic diagram illustrating another structure of a blood bag receiver of a blood irradiation apparatus in accordance with this invention.
FIG. 5 is a schematic diagram illustrating the positional relationship between the blood bag receiver and blood bags in a blood irradiation apparatus in accordance with this invention.
FIG. 6 is an illustrative flowchart of the working process of an irradiation method in accordance with this invention.

### DETAILED DESCRIPTION

The present invention will now be described in further detail in connection with the accompanying drawings and specific embodiments.

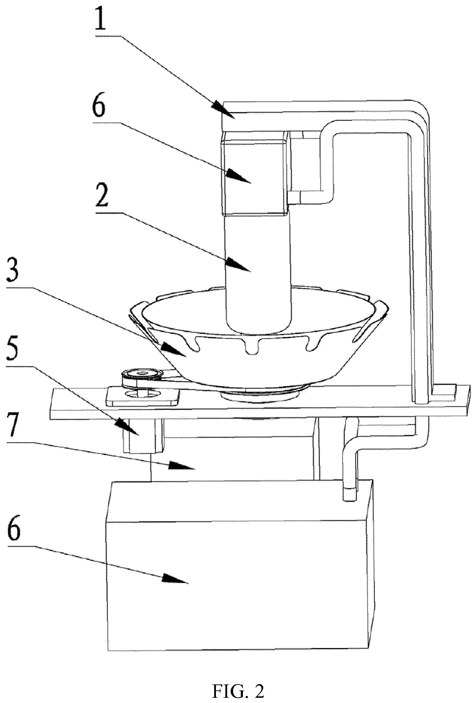

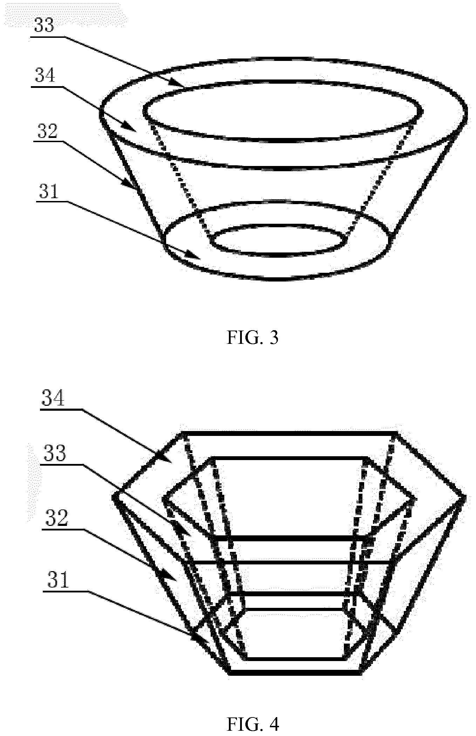

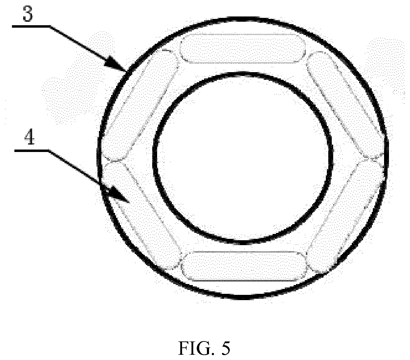

Referring to FIGS. 2 through 5, a blood irradiation apparatus with a single X-ray source according to an embodiment of this invention may include a framework 1 and an X-ray tube 2 disposed on the framework 1.

A blood bag receiver 3 may be arranged below the X-ray tube 2. The blood bag receiver 3 may be composed of a bottom plate 31, and an outer wall 32 and an inner wall 33 disposed on the bottom plate 31. An annular blood bag accommodating cavity 34 may be enclosed between the outer and inner walls 32 and 33 and may be used for placing at least one blood bag 4 to be irradiated. The blood bag 4 may be filled with blood or a blood product. The bottom plate 31 of the blood bag receiver 3 may be movably disposed on the framework 1 via a rotating shaft, and the rotating shaft may be connected with an output shaft of a rotating motor 5 via a transmission. In this invention, a plurality of blood bags 4 can be placed in the blood bag accommodating cavity 34 so that the lower part of the X-ray tube would be surrounded by a plurality of blood bags, thus fully utilizing the irradiated regions of the X-rays to improve the irradiation efficiency.

Since the blood bag 4 rotates rapidly along with the blood bag receiver 3, the blood or blood product in the blood bag receiver 3 can flow in the blood bag so that the blood or blood product in various regions can exchange their positions with one another, resulting in more uniform radiation.

Further, the outer wall 32 and the inner wall 33 may run parallel to each other, and both may be tilted relative to the bottom plate 31. The angle of tilt may be selected from the range of 0 to 90 degrees.

Further, the outer wall 32 and the inner wall 33 may be separated by a distance that matches a thickness of the blood bag 4. Typically, the distance between the outer wall 32 and the inner wall 33 may allow only one blood bag to be placed. Thus, the distance between the outer wall 32 and the inner wall 33 can be used to limit the thickness of the blood bag 4, reducing the non-uniformity of the radiation doses absorbed by the inner and outer surfaces of blood or blood products.

Further, the blood bag receiver 3 may be a truncated cone or a truncated pyramid.

Further, the inner wall 33 of the blood bag receiver 3 may be made of a radiolucent material that is permeable to X rays.

Further, the framework 1 may be provided with a dose monitoring device. The dose monitoring device may be disposed below the blood bag receiver 3 and used for detecting an absorbed dose of the blood bag 4.

Further, the framework 1 may be provided with a high-voltage power supply 7 for supplying power to the X-ray tube 2. Each of the high-voltage power supply 7 and the X-ray tube 2 may be provided with a heat dissipating device 6.

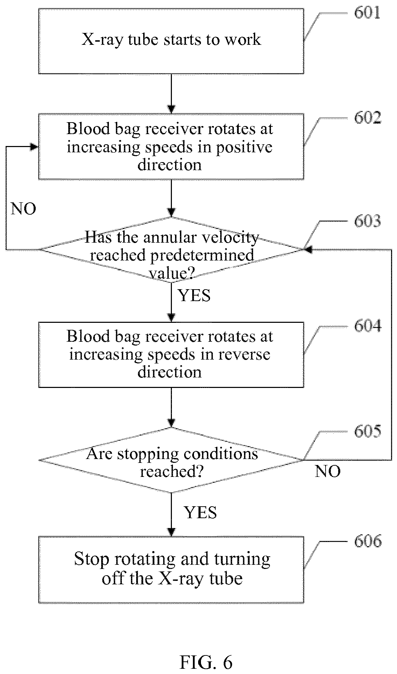

Referring now to FIG. 6, the present invention also provides an irradiation method using the above-described blood irradiation apparatus with a single X-ray source. The method may comprise the following operations.

At step 601, the X-ray tube starts to work.

After at least one blood bag is placed in the blood bag accommodating cavity, the X-ray tube may start to work.

At step 602, the blood bag receiver may rotate at increasing speeds in a positive direction.

As soon as the X-ray tube starts to work, the rotating motor may drive the blood bag receiver to rotate in a same direction at increasing speeds.

At step 603, an angular velocity is determined as to whether it has reached a predetermined value.

The instantaneous angular velocity of the blood bag receiver may be detected, and when the angular velocity of the blood bag receiver has reached a predetermined speed, the blood bag receiver may start to decelerate,

At step 604, the blood bag receiver may rotate at increasing speeds in a reverse direction.

When the angular velocity of the blood bag receiver decreases to zero, the rotating motor then may drive the blood bag receiver to rotate at increasing speeds in a direction opposite to the direction of the previous rotation, and again perform the step 603 in which the angular velocity of the blood bag receiver is detected. Therefore, multiple times of round-trip accelerated rotations may be performed,

At step 605, determination is made as to whether the stopping conditions are reached.

The predetermined stopping conditions may include that an absorbed dose of the at least one blood bag has reached a predetermined dose, or a duration of rotation has reached a predetermined duration of time, or a number of rotations has reached a predetermined number of times. Accordingly, when the above conditions are met, step 606 may be performed in which the blood bag receiver stops rotating and the X-ray tube is turned off; otherwise the current working status would be maintained, and the step 603 may continue to be performed in which the instantaneous angular velocity of the blood bag receiver is detected.

That is, in step 605, an absorbed dose of the blood bag is detected, and when the absorbed dose of the blood bag has reached a predetermined dose, the blood bag receiver is controlled to stop rotating, and the X-ray tube is controlled to turn off. Alternatively, a duration of rotation or a number of rotations of the blood bag receiver may be determined, and when the duration of rotation or the number of rotations reaches a predetermined value, the blood bag receiver may be controlled to stop rotating and the X-ray tube may be controlled to turn off.

At step 606, the blood bag receiver stops rotating and the X-ray tube is turned off.

When the predetermined stopping conditions are reached, the blood bag receiver may be controlled to stop rotating and the X-ray tube may be controlled to turn off.

According to this invention, the blood bag receiver may undergo multiple times of rapid acceleration, rapid deceleration, and reciprocating motion, and the acceleration, deceleration, and centrifugal force involved during this process can force the blood or blood product in the blood bag to flow so that the blood or blood product in various local regions can exchange their positions with one another, thereby improving the uniformity of radiation of the blood or blood product Furthermore, the rapid acceleration and deceleration can enable the blood or blood product to overcome the viscous resistance in the blood bag and thus to flow rapidly, making the radiation more uniform.

Although the present invention has been described in connection with current exemplary embodiments, those of skill in the art will appreciate that the above exemplary embodiments are merely intended for illustrating the present invention rather than limiting the scope of protection of the present invention.

## Claims

1. A blood irradiation apparatus with a single X-ray source, comprising: a framework (1), an X-ray tube (2) disposed on the framework (1), and a rotating motor having an output shaft, **characterized in that** the blood irradiation apparatus further comprises:
a blood bag receiver (3) disposed below the X-ray tube (2) and composed of a bottom plate (31) as well as an outer wall (32) and inner wall (33) that are arranged on the bottom plate (31); wherein an annular blood bag accommodating cavity (34) is enclosed between the outer and inner walls (32 and 33) and is configured for placing at least one blood bag (4) to be irradiated with each being filled with blood or a blood product, both the outer and inner walls (32 and 33) are tilted relative to the bottom plate (31), the outer wall (32) and the inner wall (33) are separated by a distance that matches a thickness of the at least one blood bag (4), and the bottom plate (31) of the blood bag receiver (3) is movably disposed on the framework (1) via a rotating shaft; wherein the rotating shaft is connected to the output shaft via a transmission.

2. The blood irradiation apparatus according to claim 1, **characterized in that** the outer wall (32) and the inner wall (33) run parallel to each other.

3. The blood irradiation apparatus according to claim 2, **characterized in that** an amount of the at least one blood bag (4) is multiple, and the multiple blood bags (4) are placed in the single blood bag accommodating cavity (34) and arranged surrounding a lower part of the X-ray tube (2).

4. The blood irradiation apparatus according to any one of the foregoing claims, **characterized in that** the blood bag receiver (3) is a truncated cone or a truncated pyramid.

5. The blood irradiation apparatus according to any one of the foregoing claims, **characterized in that** the inner wall (33) of the blood bag receiver (3) is made of a radiolucent material permeable to X rays.

6. The blood irradiation apparatus according to any one of the foregoing claims, wherein the framework (1) is provided with a dose monitoring device disposed below the blood bag receiver (3) and configured for detecting an absorbed dose of the at least one blood bag (4).

7. The blood irradiation apparatus according to any one of the foregoing claims, wherein the framework (1) is further provided with a high-voltage power supply configured for supplying power to the X-ray tube (2), and each of the high-voltage power supply and the X-ray tube (2) is provided with a heat dissipating device.

8. An irradiation method using the blood irradiation apparatus with a single X-ray source according to claim 1, **characterized in that** the method comprises:
starting to work by the X-ray tube after at least one blood bag is placed in the blood bag accommodating cavity (601);
driving, by the rotating motor, the blood bag receiver to rotate in a same direction at increasing speeds as soon as the X-ray tube starts to work (602);
detecting an instantaneous angular velocity of the blood bag receiver, and starting to decelerate by the blood bag receiver when the angular velocity of the blood bag receiver has reached a predetermined speed (603);
rotating, by the blood bag receiver, at increasing speeds in a direction opposite to the direction of the previous rotation when the angular velocity of the blood bag receiver has decreased to zero, and again detecting the instantaneous angular velocity of the blood bag receiver (604);
determining whether predetermined stopping conditions are reached (605); and
controlling the blood bag receiver to stop rotating and the X-ray tube to turn off when the predetermined stopping conditions are reached (606).

9. The irradiation method according to claim 8, **characterized in that** said controlling the blood bag receiver to stop rotating and the X-ray tube to turn off when the predetermined stopping conditions are reached (606) comprises:
detecting an absorbed dose of the at least one blood bag, and stopping the rotation of the blood bag receiver and turning off the X-ray tube when the absorbed dose of the at least one blood bag has reached a predetermined dose.

10. The irradiation method according to claim 8, **characterized in that** said controlling the blood bag receiver to stop rotating and the X-ray tube to turn off when the predetermined stopping conditions are reached (606) comprises:
detecting a duration of rotation or a number of rotations of the blood bag receiver, and controlling the blood bag receiver to stop rotating and the X-ray tube to turn off when the duration of rotation or number of rotations reaches a predetermined value.

## Patentansprüche

1. Eine Blutbestrahlungsgerät mit einer einzelnen Röntgenquelle, umfassend: ein Rahmen (1), eine Röntgenröhre (2) auf dem Rahmen (1) angeordnet, und einen rotierenden Motor mit einer Ausgangswelle, **dadurch gekennzeichnet, dass** die Blutbestrahlungsvorrichtung ferner umfasst:
eine Blutbeutelaufnahme (3), die unterhalb der Röntgenröhre (2) angeordnet ist und aus einer Bodenplatte (31) sowie einer Außenwand (32) und einer Innenwand (33) besteht die auf der Bodenplatte (31) angeordnet sind; wobei ein ringförmiger Blutbeutelaufnahmehohlraum (34) zwischen der Außenwand und der Innenwand (32 und 33) eingeschlossen ist und zum Platzieren mindestens eines zu bestrahlenden Blutbeutel (4) konfiguriert ist wobei jedes mit Blut oder einem Blutprodukt gefüllt ist; Sowohl die Außenwand auch die Innenwand (32 und 33) sind gegenüber der Bodenplatte (31) geneigt; die Außenwand (32) und die Innenwand (33) sind durch einen Abstand getrennt, der einer Dicke des mindestens einen Blutbeutels (4) entspricht, und die Bodenplatte (31) der Blutbeutelaufnahme (3) ist angeordnet beweglich auf dem Rahmen (1) über eine rotierende Welle; wobei die rotierende Welle verbunden ist zur Ausgangswelle über eine Übertragung.

2. Der Blutbestrahlungsgerät nach Anspruch 1, **dadurch gekennzeichnet, dass** die Außenwand (32) und die Innenwand (33) parallel zueinander verlaufen.

3. Der Blutbestrahlungsgerät nach Anspruch 2, **dadurch gekennzeichnet, dass** eine Menge von mindestens einem Blutbeutel (4) mehrfach vorhanden ist und die Mehrfachblutbeutel (4) in dem einzigen Blutbeutelaufnahmehohlraum (34) angeordnet sind und angeordnet um einen unteren Teil der Röntgenröhre (2).

4. Der Blutbestrahlungsgerät nach einem der vorhergehenden Ansprüche, **dadurch gekennzeichnet, dass** die Blutbeutelaufnahme (3) ein Kegelstumpf oder ein Pyramidenstumpf ist.

5. Der Blutbestrahlungsgerät nach einem der vorhergehenden Ansprüche, **dadurch gekennzeichnet, dass** die Innenwand (33) des Blutbeutelaufnehmers (3) aus einem strahlendurchlässigen Material besteht durchlässig für Röntgenstrahlen.

6. Der Blutbestrahlungsgerät nach einem der vorhergehenden Ansprüche, wobei das Rahmen (1) mit einer Dosisüberwachungsvorrichtung versehen ist, die unterhalb der Blutbeutelaufnahme (3) und angeordnet ist konfiguriert zum Erfassen einer absorbierten Dosis des mindestens einen Blutbeutel (4).

7. Der Blutbestrahlungsgerät nach einem der vorhergehenden Ansprüche, wobei das Rahmen (1) ferner mit einer Hochspannungsstromversorgung versehen ist, die zur Stromversorgung der Röntgenröhre (2) konfiguriert ist, und die Hochspannungsversorgung und die Röntgenröhre (2) jeweils mit einer Wärmeableitungsvorrichtung versehen sind.

8. Eine Bestrahlungsverfahren unter Verwendung der Blutbestrahlungsgerät mit einer einzelnen Röntgenquelle nach Anspruch 1, **dadurch gekennzeichnet, dass** das Verfahren umfasst:
Beginnen der Arbeit mit der Röntgenröhre, nachdem mindestens ein Blutbeutel in den Blutbeutelaufnahmehohlraum (601) gelegt wurde;
Antreiben, durch den rotierenden Motor, der Blutbeutelaufnahme um sich mit zunehmender Geschwindigkeit in die gleiche Richtung zu drehen, sobald die Röntgenröhre zu arbeiten beginnt (602);
Erfassen einer augenblicklichen Winkelgeschwindigkeit der Blutbeutelaufnahme und Starten einer Verzögerung durch der Blutbeutelaufnahme, wenn die Winkelgeschwindigkeit der Blutbeutelaufnahme eine vorbestimmte Geschwindigkeit erreicht hat (603);
Drehen durch den Blutbeutelaufnahme mit zunehmenden Geschwindigkeiten in einer Richtung entgegengesetzt zur Richtung der vorhergehenden Drehung, wenn die Winkelgeschwindigkeit der Blutbeutelaufnahme auf Null abgenommen hat, und erneutes Erfassen der augenblicklichen Winkelgeschwindigkeit der Blutbeutelaufnahme (604));
Bestimmen, ob vorbestimmte Stoppbedingungen erreicht sind (605); und
Steuern der Blutbeutelaufnahme, um die Drehung zu stoppen, und der Röntgenröhre, um sich auszuschalten, wenn die vorbestimmten Stoppbedingungen erreicht sind (606).

9. Bestrahlungsverfahren nach Anspruch 8, **dadurch gekennzeichnet, dass** das Steuern der Blutbeutelaufnahme zum Stoppen der Drehung und der Röntgenröhre zum Abschalten, wenn die vorbestimmten Stoppbedingungen erreicht sind (606) Folgendes umfasst:
Erfassen einer absorbierten Dosis des mindestens einen Blutbeutels und Stoppen der Drehung der Blutbeutelaufnahme und Ausschalten der Röntgenröhre, wenn die absorbierte Dosis des mindestens einer Blutbeutel eine vorbestimmte Dosis erreicht hat.

10. Bestrahlungsverfahren nach Anspruch 8, **dadurch gekennzeichnet, dass** das Steuern der Blutbeutelaufnahme zum Stoppen der Drehung und der Röntgenröhre zum Abschalten, wenn die vorbestimmten Stoppbedingungen erreicht sind (606) Folgendes umfasst:
Erfassen einer Rotationsdauer oder einer Anzahl von Rotationen der Blutbeutelaufnahme und Steuern der Blutbeutelaufnahme, um die Rotation zu stoppen, und der Röntgenröhre, um sich auszuschalten, wenn die Rotationsdauer oder die Anzahl von Rotationen einen vorbestimmten Wert erreicht.

## Revendications

1. Un appareil d'irradiation du sang avec une seule source de rayons X, comprenant: une structure (1), un tube à rayons X (2) disposé sur le bâti (1) et un moteur rotatif ayant un arbre de sortie, **caractérisé en ce que** le sang l'appareil d'irradiation comprend en outre:
un récepteur de poche de sang (3) disposé sous le tube à rayons X (2) et composé d'une plaque inférieure (31) ainsi que d'une paroi extérieure (32) et d'une paroi intérieure (33) qui sont disposées sur la plaque inférieure (31)) dans lequel une cavité annulaire de réception de poche de sang (34) est enfermée entre les parois extérieure et intérieure (32 et 33) et est conçue pour placer au moins une poche de sang (4) pour être irradiés avec chacun d'eux rempli de sang ou d'un produit sanguin, les parois externe et interne (32 et 33) sont toutes deux inclinées par rapport à la plaque inférieure (31), la paroi externe (32) et la paroi interne (33) sont séparés par une distance qui correspond à une épaisseur d'au moins une poche de sang (4), et la plaque inférieure (31) du récepteur de poche de sang (3) est disposée de manière mobile sur le bâti (1) via un arbre rotatif; dans lequel l'arbre rotatif est relié à l'arbre de sortie via une transmission.

2. L'appareil d'irradiation du sang selon la revendication 1, **caractérisé en ce que** la paroi extérieure (32) et la paroi intérieure (33) s'étendent parallèlement l'une à l'autre.

3. L'appareil d'irradiation de sang selon la revendication 2, **caractérisé en ce qu** 'une quantité d'au moins une poche de sang (4) est multiple, et les multiples poches de sang (4) sont placées dans la cavité unique de réception de poche de sang (34) et disposées entourant une partie inférieure du tube à rayons X (2).

4. L'appareil d'irradiation de sang selon l'une quelconque des revendications précédentes, **caractérisé en ce que** le récepteur de poche de sang (3) est un cône tronqué ou une pyramide tronquée.

5. L'appareil d'irradiation de sang selon l'une quelconque des revendications précédentes, **caractérisé en ce que** la paroi interne (33) du récepteur de poche de sang (3) est constituée d'un matériau radiolucent perméable aux rayons X.

6. L'appareil d'irradiation de sang selon l'une quelconque des revendications précédentes, dans lequel le bâti (1) est munie d'un dispositif de surveillance de dose disposé sous le récepteur de poche de sang (3) et configuré pour détecter une dose absorbée d'au moins une poche de sang 4)

7. L'appareil d'irradiation du sang selon l'une quelconque des revendications précédentes, dans lequel le bati (1) est en outre pourvue d'une alimentation en énergie à haute tension configurée pour alimenter en énergie le tube à rayons X (2) et chacun l'alimentation en énergie et le tube à rayons X (2) est muni d'un dispositif de dissipation de chaleur.

8. Un procédé d'irradiation utilisant l'appareil d'irradiation du sang avec une source de rayons X unique selon la revendication 1, **caractérisé en ce que** le procédé comprend:
commencer à travailler par le tube à rayons X après qu'au moins une poche de sang ait été placée dans la cavité de réception de poche de sang (601);
entraîner le récepteur de poche de sang, par le moteur en rotation, à tourner dans le même sens à des vitesses croissantes dès que le tube à rayons X commence à fonctionner (602);
détecter une vitesse angulaire instantanée du récepteur de poche de sang et commencer à décélérer par le récepteur de poche de sang lorsque la vitesse angulaire du récepteur de poche de sang a atteint une vitesse prédéterminée (603);
tourner, par le récepteur de poche de sang, à des vitesses croissantes dans une direction opposée à celle de la rotation précédente lorsque la vitesse angulaire du récepteur de poches de sang a été réduite à zéro, et détecter à nouveau la vitesse angulaire instantanée du récepteur de poche de sang (604);
déterminer si des conditions d'arrêt prédéterminées sont atteintes (605);
commander au récepteur de la poche de sang de cesser de tourner et au tube à rayons X de s'éteindre lorsque les conditions d'arrêt prédéterminées sont atteintes (606).

9. Le procédé d'irradiation selon la revendication 8, **caractérisé en ce que** ladite commande du récepteur de poche de sang pour arrêter sa rotation et le tube à rayons X pour qu'il s'éteigne lorsque les conditions d'arrêt prédéterminées sont atteintes (606) comprennent:
détecter une dose absorbée d'au moins une poche de sang, et arrêter la rotation du récepteur de poche de sang et éteindre le tube à rayons X lorsque la dose absorbée d'au moins une poche de sang a atteint une dose prédéterminée.

10. Le procédé d'irradiation selon la revendication 8, **caractérisé en ce que** ladite commande du récepteur de poche de sang pour arrêter sa rotation et le tube à rayons X pour qu'il s'éteigne lorsque les conditions d'arrêt prédéterminées sont atteintes (606) comprennent;
détecter une durée de rotation ou un nombre de rotations du récepteur de poche de sang, et commander au récepteur de poche de sang de cesser de tourner et au tube à rayons X de s'éteindre lorsque la durée de rotation ou le nombre de rotations atteint une valeur prédéterminée.
